# EUROPEAN PATENT SPECIFICATION

(11) **EP 3 505 196 B1**
(45) Date of publication and mention of the grant of the patent: **09.09.2020**
(21) Application number: 18175391.4
(22) Date of filing: 31.05.2018
(51) Int. Cl.: A61L 27/36, A61L 15/40

(54) **METHOD OF PREPARATION OF AMNIOTIC MEMBRANE-BASED MATERIAL AND A MATERIAL OBTAINABLE BY SAID METHOD**
VERFAHREN ZUR HERSTELLUNG VON MATERIAL AUF BASIS VON AMNIOTISCHER MEMBRAN UND DURCH BESAGTES VERFAHREN ERHALTENES MATERIAL
PROCÉDÉ DE PRÉPARATION D'UN MATÉRIAU À BASE DE MEMBRANE AMNIOTIQUE ET MATÉRIAU SUSCEPTIBLE D'ÊTRE OBTENU PAR CE PROCÉDÉ

(30) Priority: 27.12.2017 CZ 20170846
(43) Date of publication of application: 03.07.2019
(73) Proprietor: BioHealing k.s., 708 00 Ostrava - Poruba (CZ)
(72) Inventor: Minarikova, Iveta, 76319 Drzkova (CZ)
(74) Representative: Hartvichova, Katerina

(56) References cited:
- WO-A1-2013/095830
- WO-A2-2012/003377
- US-A1- 2016 324 797
- DATABASE WPI Week 201613 Thomson Scientific, London, GB; AN 2015-746533 XP002787366, -& CN 104 998 294 A (LUO J) 28 October 2015 (2015-10-28)
- MEHRNOOSH SAGHIZADEH ET AL: "A Simple Alkaline Method for Decellularizing Human Amniotic Membrane for Cell Culture", PLOS ONE, vol. 8, no. 11, 13 November 2013 (2013-11-13), page e79632, XP055533623, DOI: 10.1371/journal.pone.0079632
- LENKA VINKLÁRKOVÁ ET AL: "Formulation of Novel Layered Sodium Carboxymethylcellulose Film Wound Dressings with Ibuprofen for Alleviating Wound Pain", BIOMED RESEARCH INTERNATIONAL, vol. 2015, 1 January 2015 (2015-01-01), pages 1-11, XP055533626, ISSN: 2314-6133, DOI: 10.1155/2015/892671
- DATABASE WPI Week 198349 Thomson Scientific, London, GB; AN 1983-837760 XP002787370, & SU 995 718 A (ASTRAKHAN MED INST) 15 February 1983 (1983-02-15)
- P. J ADDS ET AL: "Bacterial contamination of amniotic membrane", BRITISH JOURNAL OF OPHTHALMOLOGY, vol. 85, no. 2, 1 February 2001 (2001-02-01), pages 228-230, XP055533512, GB ISSN: 0007-1161, DOI: 10.1136/bjo.85.2.228
- KATERINA JIRSOVA ET AL: "Amniotic membrane in ophthalmology: properties, preparation, storage and indications for grafting-a review", CELL AND TISSUE BANKING, vol. 18, no. 2, 2 March 2017 (2017-03-02), pages 193-204, XP055533412, NL ISSN: 1389-9333, DOI: 10.1007/s10561-017-9618-5

## Description

### Field of Art

The present invention provides a method of preparation of an amniotic membrane-based material and the material obtainable by this method. The material is especially suitable for application to chronic wounds.

### Background Art

Amnion is an inner fetal envelope that surrounds the amniotic cavity filled with amniotic fluid (i.e. amniotic fluid in which a mammalian embryo develops). The amniotic epithelium passes through the umbilical cord to the periderm.
The use of amniotic membranes for manufacture of materials suitable for reconstruction, protection and enhancement of tissue healing is known in the art. The amniotic membrane can be used as a graft or as a wound cover, for example in cornea and other tissues reconstructions. The advantage of amniotic membranes is their very low immunogenicity. EP 1604695 describes preparation of a material for reconstruction, protection and enhancement of injured cornea healing, wherein the amniotic membrane is separated from the placenta and the chorion, the epithelial layer is removed, and the amniotic membrane is lyophilized. Although the preparation of the material is carried out under sterile conditions, it still has to be sterilized in a vacuum package using radiation.
US 2007/0071740 describes amniotic membrane preparations such as extracts, gels, stroma, and mixtures thereof with other ingredients. The preparations contain components isolated from the amniotic membrane, especially by means of extraction. These preparations are suitable for treatment of wounds, inflammations and diseases associated with angiogenesis.
US 2010/104539 describes preparation of grafts containing amniotic tissue, wherein the epithelial layer has been removed. The grafts are suitable for tissue reconstruction, for example for mucous membrane reconstruction. The method of preparation involves obtaining of a placenta, removal of the chorionic tissue, removal of all epithelial cells, and uncovering of the amnion base membrane, attachment of the first membrane to the surface of a drying device, fixing of other membranes to the first membrane to form a layered material, dehydration of the graft using the drying device, packaging. The packaging is followed by sterilization using radiation.
WO 2014/195699 describes processing of an amniotic membrane in order to circumvent the need of freezing while maintaining the production of factors. This requires maintaining epithelial cells, which produce these growth factors and which are easily destroyed by freezing. Following the packaging, also this product is sterilized.
The existing methods of processing of the amniotic membrane into materials suitable for reconstruction, protection and enhancement of healing of damaged tissues, in which the structure of the membrane is maintained, always require sterilization as the last step, even if the entire process of handling the amniotic membrane is carried out under sterile conditions. Due to the nature of the material, radiation is being used for sterilization, which, however, adversely affects the resulting material and its properties because the used gamma-radiation can cause unwanted mutations and cell death. Radiation sterilization causes changes at the molecular level and thus causes substantial changes in the structure of nucleic acids, amino acids and protein complexes. Thus, changes in structure, and possibly changes in properties, of cytokines and growth factors occur, having major effects on wound healing. In addition, radiation sterilization requires special equipment.
WO2013095830 discloses methods of producing tissue grafts based on processed amnion membranes.

Therefore, the present invention aims at elimination of the need for the final radiation sterilization of the prepared material.

### Disclosure of the Invention

The invention is defined by the claims. Any subject matter falling outside the scope of the claims is provided for information purposes only.

The present invention provides a method of processing of amniotic membrane to form a material suitable for reconstruction and/or protection of tissues, and/or for enhancement of damaged tissues healing, said method starting from a placenta, and comprising the following steps:
a) separation of an amniotic membrane from a chorion,
b) purification of the amniotic membrane in saline solution and mechanical cleaning of the amniotic membrane,
c) assessment of purity of the amniotic membrane based on its color,
d) purification of the amniotic membrane in a saline solution containing an antibiotic, and subsequent reassessment of purity of the amniotic membrane based on its color,
e) applying the amniotic membrane by its epithelial side onto a textile carrier,
f) lyophilization of the resulting material obtained in step e),
g) enclosure of the lyophilized material in a sterile coating.

The placenta can be obtained from a scheduled delivery by caesarean section from healthy mothers. After removal of the placenta and informed consent of the donor, the placenta is transferred to a laboratory operating under sterile conditions. The placenta is usually enclosed in a primary sterile packaging, which is further enclosed in a container. Before any further processing, the placenta is usually checked for contamination with pathogenic microorganisms (microbiological quality control). Preferably, placentas are processed individually, i.e. placenta from only one donor is processed in one batch.

The step of separation of the amniotic membrane from the chorion (step a)) is typically performed manually by blunt dissection. Blunt dissection starts from amniotic sac incision, and continues towards the umbilical cord all over the surface of the placenta. At the point where the amnion verges into the umbilical cord, this connection is interrupted by a section, for example, using scissors or a scalpel.

The step of purification of the amniotic membrane in saline (step b) is typically performed by inserting the amniotic membrane into saline solution and optionally gently washing. Mechanical cleaning is typically performed by placing the amnion by its epithelial layer facing the working surface, and removing the residues from the side of the amnion, which was in contact with the chorion, by gently moving the thumb and index finger over it. The process of amniotic membrane purification in saline and its mechanical cleaning may be repeated in case of need, preferably it may be repeated twice or three times.

The steps of assessment of purity of the amniotic membrane based on its color (step c) and step d) is essential to maintain sufficient sterility to eliminate the need for final radiation sterilization.
The two major sources of amniotic membrane contamination are blood residues and bile acids released from meconium. Blood residues stick to the membrane during delivery or during subsequent storage before the placenta is processed. An amniotic membrane containing blood residues is pinkish to red, depending on the amount of blood residues. Contamination with bile acids released from meconium may occur in case of postterm pregnancy, or during the delivery in case of stress of the fetus and thus release of meconium into the amniotic fluid. After such contamination, the placenta or amniotic membrane has yellow-green color.
The purity assessment of the amniotic membrane based on its color in step c) is performed by determining whether its color meets the following conditions:
- from color C: 0.0000 M: 0.0000 Y: 0.0000 K: 0.0000 (transparent) to C: 0.0000 M: 0.0392 Y: 0.0392 K: 0.0000 (reddish color), the amniotic membrane is sufficiently free of blood residues and can be used in step d), i.e. inserted into an antibiotic bath, and
- from color C: 0.0000 M: 0.0000 Y: 0.0000 K: 0.0000 (transparent) to C: 0.0157 M: 0.0000 Y: 0.1490 K: 0.0000 (greenish color), the amniotic membrane is sufficiently free of bile acids and can be used in step d), i.e., inserted into an antibiotic bath.
In case the amniotic membrane does not meet the above conditions, it is repeatedly purified by the procedure of step b) - especially if the red color is too intensive; if even the repeated purification does not lead to the above mentioned conditions, the amniotic membrane cannot be used and must be disposed as bio-waste. The amniotic membrane very often cannot be used in case the green color is too intensive.
The purity assessment of the amniotic membrane based on its color in step d) is performed by determining whether its color meets the following conditions:
- from color C: 0.0000 M: 0.0000 Y: 0.0000 K: 0.0000 (transparent) to C: 0.0000 M: 0.0118 Y: 0.0118 K: 0.0000, the amniotic membrane can be further used in step e), and
- from color C: 0.0000 M: 0.0000 Y: 0.0000 K: 0.0000 (transparent) to C: 0.0039 M: 0.0000 Y: 0.0392 K: 0.0000, the amniotic membrane can be further used in step e), whereas if these conditions are not met, the amniotic membrane is further purified or discarded.

Assessment of purity based on the color of amniotic membrane can be performed using a color scale containing fields with a progressively deeper coloration from transparent to red color, and fields with a progressively deeper coloration from transparent to green color, wherein on the color scale the boundaries of the above-mentioned ranges are indicated. The area around the colored fields is transparent, allowing the scale to be attached to the membrane, thus allowing to assess the consistency or difference of the membrane color with the color of each field.

Alternatively, the purity assessment based on color of amniotic membrane can be performed using colouristic software or colouristic mobile applications, which assign a color classification to the color of the placenta, based on its photograph or its image. The color classification system is CMYK.

Color classification can be unambiguously converted between individual color systems, whereas the most commonly used color systems are CMYK (used in the present patent application for specifying the colors), RGB (not according to the invention), HEX (not according to the invention) or Pantone (not according to the invention).

The purification of the amniotic membrane in saline containing an antibiotic (in step d) is preferably performed in a bath containing an antibiotic to saline ratio of from 1: 100 to 1:10, more preferably from 1:60 to 1:40, most preferably 1:50. The preferred antibiotic is gentamicin. Preferably, the amniotic membrane is placed in this bath for at least 15 minutes, more preferably for at least 25 minutes.

The textile carrier in step e) is preferably a hydrophobic polyester textile. This textile is usually porous. The porous hydrophobic polyester textile is, for example, a commercially available material known under the trade name Sanatyl 20, which is of sufficient purity and fulfilling other criteria for medical use.

Lyophilization of the layered material is carried out under conventional conditions, i.e. under reduced pressure, e.g. from 50 to 500 mTorr (6.7 to 67 Pa), and at low temperature, for example from -40 to -80 °C. After lyophilization, the material is wrapped in a sterile coating, preferably the coating is subsequently sealed.

In any step, the membrane can be fragmented, thus split into smaller parts. In a preferred embodiment, the fragmentation may be carried out prior to application of the amniotic membrane to the textile carrier or prior to lyophilization or after lyophilization or after removal of the material from its sterile coating immediately before its actual use.

The object of the present invention is further a lyophilized material suitable for reconstruction and/or protection of tissues, and/or for enhancement of damaged tissues healing, comprising an amniotic membrane including the epithelial layer, and a textile carrier, the lyophilized material being obtainable by the method according to the present invention.

The material obtained is particularly suitable for covering of chronic wounds, but it can also be used for other applications concerning reconstruction, protection and/or healing of damaged tissues, e.g., skin, mucous membranes, cornea, etc.

The object of the present invention is also the use of a color classification means in a color system, i.e. CMYK, or RGB (not according to the invention), HEX (not according to the invention), or Pantone (not according to the invention), for assessing the purity of the amniotic membrane in the method according to the present invention.

The color classification means in the color system may be a plastic or paper color scale, containing fields with progressively deeper coloration from transparent to red and fields with progressively deeper coloration from transparent to green, wherein on the color scale the boundaries of the following ranges are indicated:
on the red part of the scale: C: 0.0000 M: 0.0000 Y: 0.0000 K: 0.0000; C: 0.0000 M: 0.0118 Y: 0.0118 K: 0.0000, and C: 0.0000 M: 0.0392 Y: 0.0392 K: 0.0000,
and
on the green part of the scale: C: 0.0000 M: 0.0000 Y: 0.0000 K: 0.0000; C: 0.0039 M: 0.0000 Y: 0.0392 K: 0.0000, and C: 0.0157 M: 0.0000 Y: 0.1490 K: 0.0000.

In a preferred embodiment, the color scale is plastic and except the fields with progressively deeper coloration it is transparent.

Alternatively, the color classification means in the color system may be a computer program or a mobile application for image processing and color classification. Preferably, the computer program or the mobile application is modified so that the output is information whether the amniotic membrane is suitable for use in step d) (purification of the amniotic membrane in saline with an added antibiotic) or for use in step e) (applying the amniotic membrane by its epithelial side onto a textile carrier).

The invention is further explained using the following examples which should not be construed as limiting the scope of the invention.

### Examples

Before the processing of placenta is started, the documentation is checked by the responsible person of the tissue establishment to release the tissue for processing. The tissue from each donor is processed in a separate batch, which ensures that the tissue is not contaminated.
The laminar box surface is prepared. All materials required for the processing are placed aseptically into the operating field. The placenta in a primary Steriking®Cover Bag is unpacked from a plastic container. Subsequently, the placenta is unpacked from its primary coating and the entire surface of the placenta is checked within microbiological quality control (using samples from the entire surface of the placenta). The samples are sent for sterility tests.

### Separation of the amniotic membrane from the chorion (step a))

After manual identification of the place of incision of the amnion, separation of the amnion from the chorion along all the circumference of the incision is performed. The two membranes are gently separated one from another, and the separation continues towards the umbilical cord along the entire surface of the placenta.

### Preparation technique: blunt preparation.

After the amnion has been separated all over the surface, and remains fixed only at the point where it verges into the umbilical cord, this connection is interrupted using scissors or scalpel.

The removed amniotic membrane is placed in a saline solution. The residua of the amniotic sac are disposed as bio-waste.

### Amniotic membrane purification (step b))

The amnion is gently washed in a prepared dose containing saline. Subsequently, the amnion is placed by its epithelial layer towards the working surface and its contact side (with the chorionic membrane) is cleaned manually. The membrane is continuously hydrated with saline during the procedure.

### Assessment of purity of the amniotic membrane based on its color (step c))

The amnion must be clean with no blood clots or other residues, it must be transparent or nearly transparent. The purity of the amnion is evaluated using a plastic color scale, containing fields with a progressively deeper color from transparent to red and from transparent to green, with the indicated range:
on the red part of the scale: C: 0.0000 M: 0.0000 Y: 0.0000 K: 0.0000 (transparent), C: 0.0000 M: 0.0118 Y: 0.0118 K: 0.0000, and C: 0.0000 M: 0.0392 Y: 0.0392 K: 0.0000,
and
on the green part of the scale: C: 0.0000 M: 0.0000 Y: 0.0000 K: 0.0000 (transparent), C: 0.0039 M: 0.0000 Y: 0.0392 K: 0.0000, and C: 0.0157 M: 0.0000 Y: 0.1490 K: 0.0000.

The amniotic membrane purity evaluation based on its color is performed by determining whether its color meets the following conditions:
- from color C: 0.0000 M: 0.0000 Y: 0.0000 K: 0.0000 (transparent) to C: 0.0000 M: 0.0392 Y: 0.0392 K: 0.0000 (reddish coloring), the amniotic membrane is sufficiently free of blood residues and can be used in step d), i.e. inserted into an antibiotic-containing bath,
   and
- from color C: 0.0000 M: 0.0000 Y: 0.0000 K: 0.0000 (transparent) to C: 0.0157 M: 0.0000 Y: 0.1490 K: 0.0000 (greenish coloring), the amniotic membrane is sufficiently free of bile acids and it can be used in step d), i.e., inserted into an antibiotic-containing bath.

If the amniotic membrane is compliant with the color scale, it is inserted into the ATB bath. If not, the pinkish or greenish color of the amniotic membrane is further cleaned or, in case of the red or green color, the amniotic membrane is discarded.

### Purification of the amniotic membrane in an antibiotic-containing solution (step d))

Gentamicin-containing saline bath is prepared, wherein the ratio of saline to gentamicin is 50: 1. Subsequently, the amniotic membrane is placed into the prepared bath for 30 minutes.

### Assessment of the purity of the amniotic membrane based on its color (step d))

In an antibiotic bath, the color of the amniotic membrane changes and therefore it needs to be reassessed. The purity of the amnion is evaluated using a plastic color scale containing fields with a progressively deeper color from transparent to red and from transparent to green, with the following ranges being indicated on the scale:
on the red part of the scale: C: 0.0000 M: 0.0000 Y: 0.0000 K: 0.0000 (transparent), C: 0.0000 M: 0.0118 Y: 0.0118 K: 0.0000, and C: 0.0000 M: 0.0392 Y: 0.0392 K: 0.0000,
and
on the green part of the scale: C: 0.0000 M: 0.0000 Y: 0.0000 K: 0.0000 (transparent); C: 0.0039 M: 0.0000 Y: 0.0392 K: 0.0000, and C: 0.0157 M: 0.0000 Y: 0.1490 K: 0.0000.

The amniotic membrane purity evaluation based on its color is performed by determining whether its color meets the following conditions:
- from color C: 0.0000 M: 0.0000 Y: 0.0000 K: 0.0000 (transparent) to C: 0.0000 M: 0.0118 Y: 0.0118 K: 0.0000, the amniotic membrane is sufficiently free of blood residues and can be used for further processing in step e),
   and
- from color C: 0.0000 M: 0.0000 Y: 0.0000 K: 0.0000 (transparent) to C: 0.0039 M: 0.0000 Y: 0.0392 K: 0.0000, the amniotic membrane can be used for further processing in step e).

If the amniotic membrane is compliant with the color scale, it is used for further processing. If not, the pinkish or greenish color of the amniotic membrane is further cleaned or, in case of the red or green color, the amniotic membrane is discarded as bio-waste. *Membrane fragmentation and application to Sanatyl 20 (hydrophobic porous polyester textile) (step e))*

The entire amnion is resected with scissors or scalpel into several larger fragments (about 15x15cm). Sanatyl 20 is laid on the epithelial side of these fragments. Sanatyl 20 is gently pressed towards the amnion to adhere to the amnion all over the surface.

The amniotic membrane on Sanatyl 20 is inserted into plastic plates, closed and sealed into the Steriking Coverbag, frozen at -80 °C and stored at the same temperature; or directly lyophilized.

### Lyophilization (step f))

The amniotic membrane on the Sanatyl 20 carrier on plastic plates is placed into a lyophilizer. Lyophilization takes place according to the following procedure: The initial temperature of the lyophilizer is 20 °C, then the temperature drops to -45 °C during 495 minutes at the pressure of 400 mTorr (53 Pa). Subsequently, the temperature is raised to - 20 °C for 1160 minutes at the pressure of 100 mTorr (13 Pa). In the final stage of the lyophilization, the amniotic membrane is dried at the temperature of 10 °C and the pressure of 450 mTorr (60 Pa) for further 30 minutes. Then the lyophilization is terminated.

The condenser temperature is at -65 °C during the whole lyophilization procedure. After lyophilization, the lyophilized amniotic membrane on the Sanatyl 20 carrier is placed back to the plastic plates and sealed into the Steriking cover bag.

The amniotic membrane can be immediately fragmented or stored at room temperature.

## Claims

1. A method of processing of amniotic membrane to form materials suitable for reconstruction and/or protection of tissues, and/or for enhancement of damaged tissues healing, said method starting from placenta, **characterized in that** it comprises the following steps:
a) separation of an amniotic membrane from a chorion,
b) purification of the amniotic membrane in saline solution and mechanical cleaning of the amniotic membrane,
c) assessment of purity of the amniotic membrane based on its color, which is carried out by determining whether its color meets the following conditions:
- from color C: 0.0000 M: 0.0000 Y: 0.0000 K: 0.0000 to C: 0.0000 M: 0.0392 Y: 0.0392 K: 0.0000, the amniotic membrane is sufficiently free of blood residues and can be used in step d), therefore it can be placed in saline containing an antibiotic,
and
- from color C: 0.0000 M: 0.0000 Y: 0.0000 K: 0.0000 to C: 0.0157 M: 0.0000 Y: 0.1490 K: 0.0000 the amniotic membrane is sufficiently free of bile acids and can be used in step d), therefore it can be placed in saline containing an antibiotic,
whereas if these conditions are not met, the amniotic membrane is further purified or discarded;
d) purification of the amniotic membrane in a saline solution containing an antibiotic, and subsequent reassessment of purity of the amniotic membrane based on its color, which is carried out by determining whether its color meets the following conditions:
- from color C: 0.0000 M: 0.0000 Y: 0.0000 K: 0.0000 to C: 0.0000 M: 0.0118 Y: 0.0118 K: 0.0000, the amniotic membrane may further be used in step e),
and
- from color C: 0.0000 M: 0.0000 Y: 0.0000 K: 0.0000 to C: 0.0039 M: 0.0000 Y: 0.0392 K: 0.0000, the amniotic membrane may further be used in step e),
whereas if these conditions are not met, the amniotic membrane is further purified or discarded;
e) applying the amniotic membrane by its epithelial side onto a textile carrier,
f) lyophilization of the resulting material obtained in step e),
g) enclosure of the lyophilized material in a sterile coating.

2. The method according to claim 1, **characterized in that** the purity assessment based on the color of the amniotic membrane is carried out using a color scale, comprising fields with a progressively deeper coloration from transparent to red color, and fields with a progressively deeper coloration from transparent to green color,
wherein on the color scale the boundaries of the following ranges are indicated:
on the red part of the scale: C: 0.0000 M: 0.0000 Y: 0.0000 K: 0.0000; C: 0.0000 M: 0.0118 Y: 0.0118 K: 0.0000, and C: 0.0000 M: 0.0392 Y: 0.0392 K: 0.0000,
and
on the green part of the scale: C: 0.0000 M: 0.0000 Y: 0.0000 K: 0.0000; C: 0.0039 M: 0.0000 Y: 0.0392 K: 0.0000, and C: 0.0157 M: 0.0000 Y: 0.1490 K: 0.0000.

3. The method according to claim 1, **characterized in that** the purity assessment based on the color of the amniotic membrane is carried out by means of a computer program or of a mobile application for image processing and color classification, which assigns color classification to a color in a color classification system, preferably in a CMYK system.

4. The method according to any one of the preceding claims, **characterized in that** the antibiotic in step d) is gentamicin and/or the textile carrier in step e) is a hydrophobic polyester textile.

5. A lyophilized material suitable for reconstruction and/or protection of tissues, and/or for enhancement of damaged tissues healing, comprising an amniotic membrane including the epithelial layer, and a textile carrier, the lyophilized material being obtainable by the method according to any one of the preceding claims.

6. The material according to claim 5 for use for covering chronic wounds.

7. Use of a color classification means in a color system for assessing the purity of the amniotic membrane in the method according to any one of claims 1 to 4.

8. Use according to claim 7, wherein the color classification means in the color system are selected from:
- plastic or paper colored scales, containing fields with progressively deeper coloration from transparent to red and progressively deeper coloration from transparent to green, wherein on the color scale the boundaries of the following ranges are indicated:
on the red part of the scale: C: 0.0000 M: 0.0000 Y: 0.0000 K: 0.0000; C: 0.0000 M: 0.0118 Y: 0.0118 K: 0.0000, and C: 0.0000 M: 0.0392 Y: 0.0392 K: 0.0000,
and
on the green part of the scale: C: 0.0000 M: 0.0000 Y: 0.0000 K: 0.0000; C: 0.0039 M: 0.0000 Y: 0.0392 K: 0.0000, and C: 0.0157 M: 0.0000 Y: 0.1490 K: 0.0000;
- a computer program or a mobile application for image processing and color classification, preferably the computer program or the mobile application is modified so that the output is information whether the amniotic membrane is suitable for use in step d) or for use in step e).

## Patentansprüche

1. Verfahren zur Verarbeitung der Amnionmembran unter Bildung von Materialien, die zur Rekonstruktion und/oder zum Schutz von Geweben und/oder zur Verbesserung der Heilung geschädigter Gewebe geeignet sind, wobei das Verfahren ausgehend von der Plazenta **dadurch gekennzeichnet ist, dass** es die folgenden Schritte umfasst:
a) Trennung einer Amnionmembran von einem Chorion,
b) Reinigung der Amnionmembran in physiologischer Lösung und mechanische Reinigung der Amnionmembran,
c) Beurteilung der Reinheit der Amnionmembran anhand ihrer Farbe, wobei bestimmt wird, ob ihre Farbe die folgenden Bedingungen erfüllt:
- von Farbe C: 0,0000 M: 0,0000 Y: 0,0000 K: 0,0000 bis Farbe C: 0,0000 M: 0,0392 Y: 0,0392 K: 0,0000 ist die Amnionmembran ausreichend frei von Blutresten und kann in Schritt d) verwendet werden, d.h. in eine physiologische Lösung gegeben werden, die ein Antibiotikum enthält,
und
- von Farbe C: 0,0000 M: 0,0000 Y: 0,0000 K: 0,0000 bis Farbe C: 0,0157 M: 0,0000 Y: 0,1490 K: 0,0000 ist die Amnionmembran ausreichend frei von Gallensäuren und kann in Schritt d) verwendet werden, d.h. in eine physiologische Lösung gegeben werden, die ein Antibiotikum enthält,
worin wenn diese Bedingungen nicht erfüllt sind, wird die Amnionmembran weiter gereinigt oder verworfen,
d) Reinigung der Amnionmembran in einer physiologischer Lösung, die ein Antibiotikum enthält, und anschließende Neubewertung der Reinheit der Amnionmembran anhand ihrer Farbe, wobei bestimmt wird, ob ihre Farbe die folgenden Bedingungen erfüllt:
- von Farbe C: 0,0000 M: 0,0000 Y: 0,0000 K: 0,0000 bis Farbe C: 0,0000 M: 0,0118 Y: 0,0118 K: 0,0000 kann die Amnionmembran in Schritt e) weiter verwendet werden,
und
- von Farbe C: 0,0000 M: 0,0000 Y: 0,0000 K: 0,0000 bis Farbe C: 0,0039 M: 0,0000 Y: 0,0392 K: 0,0000 kann die Amnionmembran in Schritt e) weiter verwendet werden,
worin wenn diese Bedingungen nicht erfüllt sind, wird die Amnionmembran weiter gereinigt oder verworfen,
e) Aufbringen der Amnionmembran an ihrer Epithelseite auf einen Textilträger,
f) Lyophilisierung des in Schritt e) erhaltenen resultierenden Materials,
g) Einschließen des lyophilisierten Materials in eine sterile Beschichtung.

2. Verfahren nach Anspruch 1, **dadurch gekennzeichnet, dass** die Reinheitsbewertung anhand der Farbe der Amnionmembran unter Verwendung einer Farbskala durchgeführt wird, die Felder mit einer zunehmend tieferen Färbung von transparenter zu roter Farbe und Felder mit einer zunehmend tieferen Färbung von transparenter zu grüner Farbe umfasst,
wobei auf der Farbskala die Grenzen der folgenden Bereiche angegeben sind:
auf dem roten Teil der Skala: C: 0,0000 M: 0,0000 Y: 0,0000 K: 0,0000; C: 0,0000 M: 0,0118 Y: 0,0118 K: 0,0000 und C: 0,0000 M: 0,0392 Y: 0,0392 K: 0,0000;
und
auf dem grünen Teil der Skala: C: 0,0000 M: 0,0000 Y: 0,0000 K: 0,0000; C: 0,0039 M: 0,0000 Y: 0,0392 K: 0,0000 und C: 0,0157 M: 0,0000 Y: 0,1490 K: 0,0000.

3. Verfahren nach Anspruch 1, **dadurch gekennzeichnet, dass** die Reinheitsbewertung anhand der Farbe der Amnionmembran mittels eines Computerprogramms oder einer mobilen Applikation zur Bildverarbeitung und Farbklassifizierung durchgeführt wird, die für eine Farbe eine Farbklassifizierung zuordnet in einem Farbklassifizierungssystem, vorzugsweise in einem CMYK-System.

4. Verfahren nach einem der vorhergehenden Ansprüche, **dadurch gekennzeichnet, dass** das Antibiotikum in Schritt d) Gentamicin ist und/oder der Textilträger in Schritt e) ein hydrophobes Polyestertextil ist.

5. Lyophilisiertes Material, das zur Rekonstruktion und/oder zum Schutz von Geweben und/oder zur Verbesserung der Heilung geschädigter Gewebe geeignet ist, umfassend eine Amnionmembran einschließlich der Epithelschicht und einen Textilträger, wobei das lyophilisierte Material durch das Verfahren nach einem der vorhergehenden Ansprüche erhältlich ist.

6. Material nach Anspruch 5 zur Verwendung zum Abdecken chronischer Wunden.

7. Verwendung eines Farbklassifizierungsmittels in einem Farbsystem zur Beurteilung der Reinheit der Amnionmembran in dem Verfahren nach einem der Ansprüche 1 bis 4.

8. Verwendung nach Anspruch 7, wobei die Farbklassifizierungsmittel ausgewählt sind aus:
- farbende Plastik- oder Papierskalen, die Felder mit einer zunehmend tieferen Färbung von transparent nach rot und einer zunehmend tieferen Färbung von transparent nach grün enthalten, wobei auf der Farbskala die Grenzen der folgenden Bereiche angegeben sind:
auf dem roten Teil der Skala: C: 0,0000 M: 0,0000 Y: 0,0000 K: 0,0000; C: 0,0000 M: 0,0118 Y: 0,0118 K: 0,0000 und C: 0,0000 M: 0,0392 Y: 0,0392 K: 0,0000;
und
auf dem grünen Teil der Skala: C: 0,0000 M: 0,0000 Y: 0,0000 K: 0,0000; C: 0,0039 M: 0,0000 Y: 0,0392 K: 0,0000 und C: 0,0157 M: 0,0000 Y: 0,1490 K: 0,0000;
- ein Computerprogramm oder eine mobile Applikation zur Bildverarbeitung und Farbklassifizierung, vorzugsweise das Computerprogramm oder die mobile Applikation so modifiziert ist, dass die Ausgabe Informationen darüber enthält, ob die Amnionmembran zur Verwendung in Schritt d) oder zur Verwendung in Schritt e) geeignet ist.

## Revendications

1. Procédé de traitement de membrane amniotique pour former des matériaux pour la reconstruction et/ou à la protection des tissus, et/ou à l'amélioration de la guérison des tissus endommagés, ledit procédé partant du placenta, **caractérisé en ce qu'**il comprend les étapes suivantes:
a) séparation d'une membrane amniotique d'un chorion,
b) purification de la membrane amniotique en solution saline et nettoyage mécanique de la membrane amniotique,
c) évaluation de la pureté de la membrane amniotique en fonction de sa couleur, qui est effectuée en déterminant si sa couleur remplit les conditions suivantes:
- de la couleur C: 0,0000 M: 0,0000 Y: 0,0000 K: 0,0000 à la couleur C: 0,0000 M: 0,0392 Y: 0,0392 K: 0,0000, la membrane amniotique est suffisamment purifiée des résidus sanguins et peut être utilisée à l'étape d), elle peut donc être placé dans une solution saline contenant un antibiotique,
et
- de la couleur C: 0,0000 M: 0,0000 Y: 0,0000 K: 0,0000 à la couleur C: 0,0157 M: 0,0000 Y: 0,1490 K: 0,0000 la membrane amniotique est suffisamment purifiée des acides biliaires et peut être utilisée à l'étape d), elle peut donc être placé dans une solution saline contenant un antibiotique,
où si ces conditions ne sont pas remplies, la membrane amniotique est davantage purifiée ou éliminée;
d) purification de la membrane amniotique dans une solution saline contenant un antibiotique et réévaluation ultérieure de la pureté de la membrane amniotique en fonction de sa couleur, qui est effectuée en déterminant si sa couleur remplit les conditions suivantes:
- de la couleur C: 0,0000 M: 0,0000 Y: 0,0000 K: 0,0000 à la couleur C: 0,0000 M: 0,0118 Y: 0,0118 K: 0,0000, la membrane amniotique peut être utilisée à l'étape e),
et
- de la couleur C: 0,0000 M: 0,0000 Y: 0,0000 K: 0,0000 à la couleur C: 0,0039 M: 0,0000 Y: 0,0392 K: 0,0000, la membrane amniotique peut être utilisée à l'étape e),
où si ces conditions ne sont pas remplies, la membrane amniotique est davantage purifiée ou éliminée;
e) appliquer la membrane amniotique par sa face épithéliale sur un support textile,
f) lyophilisation du matériau obtenu à l'étape e),
g) enfermement du matériau lyophilisé dans un revêtement stérile.

2. Procédé selon la revendication 1, **caractérisé en ce que** l'évaluation de la pureté en fonction de la couleur de la membrane amniotique est réalisée à l'aide d'une échelle de couleurs, comprenant des champs avec une coloration progressivement plus profonde de la couleur transparente à la couleur rouge, et des champs avec une coloration progressivement plus profonde de la couleur transparente à la couleur verte,
où sur l'échelle de couleurs les limites des plages suivantes sont indiquées:
sur la partie rouge de l'échelle: C: 0,0000 M: 0,0000 Y: 0,0000 K: 0,0000; C: 0,0000 M: 0,0118 Y: 0,0118 K: 0,0000, et C: 0,0000 M: 0,0392 Y: 0,0392 K: 0,0000,
et
sur la partie verte de l'échelle: C: 0,0000 M: 0,0000 Y: 0,0000 K: 0,0000; C: 0,0039 M: 0,0000 Y: 0,0392 K: 0,0000 et C: 0,0157 M: 0,0000 Y: 0,1490 K: 0,0000.

3. Procédé selon la revendication 1, **caractérisé en ce que** l'évaluation de la pureté en fonction de la couleur de la membrane amniotique est réalisée au moyen d'un programme informatique ou d'une application mobile de traitement d'image et de classification des couleurs, qui attribue à un couleur une classification de couleur dans un système de classification des couleurs, de préférence dans un système CMYK.

4. Procédé selon l'une quelconque des revendications précédentes, **caractérisé en ce que** l'antibiotique de l'étape d) est la gentamicine et/ou le support textile de l'étape e) est un textile polyester hydrophobe.

5. Matériau lyophilisé pour la reconstruction et/ou la protection des tissus, et/ou pour l'amélioration de la guérison des tissus endommagés, comprenant une membrane amniotique comprenant la couche épithéliale, et un support textile, le matériau lyophilisé pouvant être obtenu par le procédé selon l'une quelconque des revendications précédentes.

6. Matériau selon la revendication 5 pour utilisation pour couvrir des plaies chroniques.

7. Utilisation d'un moyen de classification de couleur dans un système de couleur pour évaluer la pureté de la membrane amniotique dans le procédé selon l'une quelconque des revendications 1 à 4.

8. Utilisation selon la revendication 7, où les moyens de classification de couleur dans le système de couleur sont choisis parmi:
- des échelles colorées en plastique ou en papier, contenant des champs avec une coloration progressivement plus profonde du transparent au rouge et une coloration progressivement plus profonde du transparent au vert, où sur l'échelle de couleur les limites des plages suivantes sont indiquées:
sur la partie rouge de l'échelle: C: 0,0000 M: 0,0000 Y: 0,0000 K: 0,0000; C: 0,0000 M: 0,0118 Y: 0,0118 K: 0,0000, et C: 0,0000 M: 0,0392 Y: 0,0392 K: 0,0000,
et
sur la partie verte de l'échelle: C: 0,0000 M: 0,0000 Y: 0,0000 K: 0,0000; C: 0,0039 M: 0,0000 Y: 0,0392 K: 0,0000 et C: 0,0157 M: 0,0000 Y: 0,1490 K: 0,0000;
- un programme informatique ou une application mobile pour le traitement d'image et la classification des couleurs, de préférence le programme informatique ou l'application mobile est modifié de sorte que la sortie soit une information indiquant si la membrane amniotique peut être utilisée à l'étape d) ou à l'étape e) .
